# EUROPEAN PATENT APPLICATION

(11) **EP 1 431 399 A1**
(43) Date of publication of application: **23.06.2004**
(21) Application number: 02293199.2
(22) Date of filing: 20.12.2002
(51) Int. Cl.: C12Q 1/68, A61K 45/06

(54) **Methods and composition for identifying therapeutic agents of atherosclerotic plaque lesions**

(71) Applicant: Clinigenetics, 30000 Nîmes (FR)
(72) Inventor: Marguerie, Gérard, 94400 VITRY-SUR-SEINE (FR)
(74) Representative: Breesé, Pierre

(57) **Abstract**

The present invention relates to a method for monitoring the growth, erosion, rupture or stability of an atherosclerotic plaque comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

## Description

### Field of the invention

The invention pertains to the field of methods and compositions for monitoring the growth, erosion, rupture or stability of an atherosclerotic plaque. The invention also relates to methods and compositions for identifying therapeutic agents useful in humans for the treatment of atherosclerotic lesions in relation with the growth, erosion and rupture of an arterial plaque.

The present invention is based on the observation that a plurality of genes are differentially expressed in an atherosclerotic plaque relative to their normal expression and are coexpressed with atherosclerosis associated genes under conditions of hyperlipidemia and in the absence of high level of blood glucose and insulin. These genes identify new pathways and exhibit target and/or marker gene characteristics for controlling plaque development at vascular sites that are prone to atherosclerosis.

### Background of the invention

Atherosclerosis is the most important cause of cardiovascular diseases and deaths in the industrialised countries (Ross. R. 1993, Nature, 362, 801-809). Coronary atherosclerosis is responsible for over 500 000 deaths annually in the United States and for a vast number of other clinical complications.

Atherosclerosis is the result of a complex unbalanced cellular and molecular reaction which normally functions as a defense mechanism in response to vascular injury. In pathological situations, however, this mechanism leads to endothelium dysfunction, cellular changes in the arterial intima and the continuous formation and growth of an arterial plaque containing lipids and foam cells.

Mechanisms controlling plaque growth and erosion and plaque rupture leading to thrombosis are largely unknown, and there is an unmet need for drugs in this area. The process appears to be the result of conflicting mechanisms. This includes for instance, lipid deposition and removal, cellular survival and death, cellular adhesion and extracellular matrix degradation and motility.

Atherosclerosis is initiated at specific sites by endothelium injury and dysfunction. Production of oxidized lipoproteins (oxLDL) and other oxidative and cytotoxic agents is probably the initial event that causes vascular injury. These agents have been shown to stimulate both survival and pro apoptotic mechanisms in endothelial cells and macrophages. These initial reactions occur during hyperlipidemia, dyslipidemia, hypertension, diabetes and fluctuating shear stress.

Endothelial dysfunction creates a chronic inflammation which results in a continuous recruitment of monocyte and macrophages. While beneficial in normal circumstances, this phenomenon may become pathologic and contribute to arterial plaque destabilization. The process is a slow reaction when compared to monocyte recruitment during infection, and may last a life time period. Activated endothelial cells and monocytes express scavenger receptors such as CD36 or LOX1 that bind and uptake modified LDL. This reaction leads to the formation of foam cells, destabilization of the arterial plaque and causes plaque rupture resulting in acute thrombosis.

The existence of endothelium dysfunction and the perpetuation of lipid deposition and foam cells accumulation are the most important consequences of vascular lesions in patients at risk. Particularly, the abundance of oxLDL is an important factor of atherosclerosis.

This can be controlled by modulating the activity of enzymes that are involved in cholesterol synthesise to reduce the accumulation of LDL and the toxic effect of oxLDL. Inhibitors that control the HMG-CoA reductase, exemplify and support this concept. These inhibitors have successfully been used for treating atherosclerosis. Only 30% of the patients, however, were shown to be responsive, and potential side effects were observed suggesting that individual dosage is probably a critical parameter.

Many of the drugs that are directed against these enzymatic pathways intend to treat the causes of atherosclerosis. But there is a need for drugs that can treat the consequences of atherosclerosis by controlling plaque growth and stability.

The development of an arterial plaque is complex and requires the expression of many genes with multiple functions. The genes may be directly or indirectly involved in the process and may also be expressed in tissues other than vascular cells. To exhibit target characteristics, the gene must be directly involved in the pathogenesis of atherosclerosis.

### Summary of the invention

The invention is based on the discovery that Stearoyl CoA deasturase, Aldose reductase and aldehyde reductase, sphingomyelinase, acid ceramidase, Ceramide glucosyl transferase, sphingosin phosphate liase, thymosine beta 4, aldehyde dehydrogenase, ATP ase Ca++ binding protein and CD163 are up regulated in vivo in early atherosclerotic plaques both at the RNA and protein levels, and are co-expressed with genes that are known to be directly involved in the process of human atherosclerosis.

The present invention provides methods and compositions for monitoring the growth, erosion, rupture or stability of an atherosclerotic plaque. The methods involve the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

The present invention also provides a diagnostic method of atherosclerosis or cardiovascular disorders relating to the atherosclerotic plaque in a biological sample of a subject comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

The present invention relates to methods and compositions for identifying therapeutic agents useful in humans for the treatment of atherosclerotic lesions in relation with the growth, erosion and rupture of an arterial plaque. The methods involve the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, in the presence of a test compound.

Such compounds are useful to monitor the progression or regression of the atherosclerotic plaque and to inhibits the accumulation of macrophages foam cells at sites of vascular lesions when large amount of LDL and oxLDL are present. Therefore, the invention relates to the use of a compound modulating the expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, or modulating the activity of said at least one protein for the preparation of a pharmaceutical composition useful for preventing and/or treating artherosclerosis or cardiovascular disorders relating to the atherosclerotic plaque.

### Brief description of the figures

Figure 1 shows cross-sections through the left anterior descending (LAD) coronary aorta root of pigs under control and 4% cholesterol rich diet conditions. Sections A to F are representative of advanced atherosclerotic plaque at 12 weeks (A,C,E, x 10; B,D,F, x 40). Lipids were stained with oil red'O , (A and B), cells were labelled with toluidine blue ( C,D,E,F )
Figure 2 shows the plasma lipoprotein profile of hyperlipidemic pigs fed with a 4% cholesterol diet for 12 weeks. Data represent mean value for 10 pigs
Figure 3 shows laser microdissected sections from early advanced plaques and RNA ectraction from hypercholesterolemic pig. A : Section from LAD ; B : Microdissected section ; C : RNA extraction and analysis showing a high quality ratio between 18S and 28S fractions.
Figure 4 shows the amplification of mRNA from laser microdissected sections of the plaque. Panel A, Antisens RNA were amplified by two round of in vitro transcription. The factor of amplification was around 80 000. Panel B , Medium size of RNA was about 1400 nucleotides. Panel C, Linearity of this amplification reaction was estimated , using RT-PCR amplification of low, medium and high activity genes
Figure 5 shows a typical expression signature on a human DNA chip containing 12 000 different genes probes.
Figure 6 shows prototype permanent cell line with a foam cell phenotype
Figure 7 shows inhibition of vesicles accumulation in a typical foam cell using inhibitor of at least one of the protein.

### DETAILED DESCRIPTION

The present invention provides a series of genes, hereafter also call "new genes" that are differentially expressed relative to their normal expression in early and advanced atherosclerotic plaques containing macrophages, under hyperlypidemic conditions and in the absence of high levels of blood glucose and insulin. These genes identify new pathways and exhibit target and/or marker gene characteristics for controlling plaque development at vascular sites that are prone to atherosclerosis.

The present invention provides methods and compositions for controlling atherosclerotic plaque progression and erosion, and their clinical complications. The invention is based on the discovery that Stearoyl CoA deasturase, Aldose reductase and aldehyde reductase, sphingomyelinase, acid ceramidase, Ceramide glucosyl transferase, sphingosin phosphate liase, thymosine beta 4, aldehyde dehydrogenase, ATP ase Ca++ binding protein and CD163 are up regulated in vivo in early atherosclerotic plaques both at the RNA and protein levels, and are co-expressed with genes that are known to be directly involved in the process of human atherosclerosis.

The present invention relates to methods and compositions to monitor the progression or the regression of plaques and to inhibits the accumulation of macrophages foam cells at sites of vascular lesions when large amount of LDL and ox LDL are present. The method comprises the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

Said analysis is carried out in human or animal cells, tissue sections or animal models.

Discussed below are methods for prognostic and diagnostic evaluation of atherosclerosis, including the identification of subjects exhibiting a predisposition to atherosclerosis and the imaging of an atherosclerotic plaque. The invention provides a diagnostic method of artherosclerosis or cardiovascular disorders relating to the atherosclerotic plaque in a biological sample of a subject comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

Said analysis is carried out in human or animal cells or tissue sections.

According to an other embodiment, the method of the invention comprises :
- providing a plurality of different ligands in the form of an array on a solid surface, said different ligands being complementary to different segments of at least one protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, or being complementary to different segments of at least one gene coding said proteins,
- applying a sample solution potentially containing the targets of the ligands to the array of ligands under conditions which allow the interaction of said ligands and its target, and
- measuring the interactions of the targets with the different ligands of the array.

In preferred embodiments, the ligands are nucleic acid probes and the sample contains target nucleic acids in order to measure the hybridation of the probes with the target nucleic acids. Avantagously, the nucleic acid probes are oligonucleotides.

Additional embodiments of the invention provides array comprising 2 to about 200 oligonucleotides localized in discrete location per square centimeter on the solid surface.

The sample is for example from a patient developing artherosclerotic plaque.

The methods of the invention comprises the measure of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, and the comparison of said measure with the normal expression of said protein in early and advanced atherosclerotic plaques containing macrophages, under hyperlypidemic conditions and in the absence of high levels of blood glucose and insulin.

The new genes are coexpressed with reference genes known to be differentially expressed during the progression of atherosclerotic plaques in mammals and humans. According to the present invention, these reference genes are utilised as canonical genes to profile the degree of progression of the plaque.

Canonical genes refer to a set of genes that have been described to be expressed in human atherosclerotic plaque. This set of genes defines a canonical response profile and a typical signature for an atherosclerotic target. For each of the known target genes, an average of the fold changes was evaluated. Novel genes, associated with the development of an atherosclerotic plaque were characterized in reference to this set of genes and exhibited expression patterns similar to these canonical genes an were significantly and statistically differentially induced when compared with genes from non atherosclerotic vascular endothelium located at the same position in the coronary artery or non stimulated circulating monocytes.

The set of canonical genes that are representative of an atherosclerotic plaque includes but is not limited to : membrane associated genes such as CD68, CD36 which are both markers of the macrophage lineage; PECAM 1, a marker for endothelial cells; markers of the inflammatory response such as TLR4 , HSP60 and HSP70, Galectin 3 and IL1-R; markers of the oxidative stress including HIF-1 and Paraoxanase 3, metabolic marker such as NADH dehydrogenase; lipoprotein receptors such as LDL-R and VLDL-R.

Also discussed below are methods for detecting agents and associated genes that may control the activity of these proteins at sites of an atherosclerotic lesion.

Therefore, the invention relates to method of screening compounds useful for the treatment of artherosclerosis or cardiovascular disorders relating to the atherosclerotic plaque comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, in the presence of a test compound.

Said analysis is carried out in human or animal tissue sections or animal models. It can be also performed on a solid support for high throughput methods. In such embodiments, the invention comprises :
- providing a plurality of different ligands in the form of an array on a solid surface, said different ligands consisting of all or part of at least one protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, ,
- applying a solution containing a test compound to the array of ligands, and
- measuring the interaction, such as the binding, of the test compound with the different ligands of the array.

The test compounds may be proteins or molecule of small molecular weight.

The analysis according to the above methods of the present invention may be performed at the mRNA or protein level.

A method of screening compounds useful for the treatment of artherosclerosis or cardiovascular disorders relating to the atherosclerotic plaque, accoring to the present invention comprises :
- providing an assay for at least one protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, in the presence of a test compound.
- contacting said assay with a test compound, and
- measuring the action of the test compound on the said protein in the assay.

The new genes identify also new ways to treat patients with hypercholesterolemia induced atherosclerotic plaques in the absence of high blood glucose and high blood insulin levels. Therefore the invention relates to the use of a compound modulating the expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, or modulating the activity of said at least one protein for the preparation of a pharmaceutical composition useful for preventing and/or treating artherosclerosis or cardiovascular disorders relating to the atherosclerotic plaque.

### 1) Identification of at least one of the protein in atherosclerotic plaque

Differential expression refers to both, quantitative and qualitative differences in at least one of the protein mRNA and protein expression using vascular tissues containing atherosclerotic lesions. The gene may be activated or down regulated in normal vessel wall versus atherosclerotic plaque. Differential expression may be detected via differential techniques, including RT-PCR, northern analysis, DNA micro-arrays and DNA chips, differential expression libraries, immuno-histochemistry, two dimension electrophoresis, and mass spectroscopy. Differential expression also refers to expression that can be used as part of prognostic or diagnostic tools that may be useful to monitor the development of an arterial plaque in atherosclerosis.

At least one of the protein can be used as target gene. This refers to a differential expression involved in atherosclerosis in a manner that can modulate the level of gene expression or activity to modulate and ameliorate the stability of an arterial plaque. This method can be applied in different experimental paradigms such as those described below :
- **Foam Cells :** Gene differential expression or protein activity of at least one of the proteins may be used to quantitatively or qualitatively detect genes as secondary targets that are co-regulated during the maturation of macrophages and the formation of foam cells under circumstances that mimic the development of an atherosclerotic plaque. This may include for instance, but is not limited to, the presence of Lipoproteins and modified lipoproteins or components from hyperlipidemic serum. Differential expression of at least one of the protein may be used to validate an *ex vivo* model.
- **Endothelial dysfunction :** Endothelial cell monolayer can be used to monitor gene expression or protein activity that may be correlated with a differential expression and activity of at least one of the protein and may have target characteristics under circumstances that mimic atherosclerosis. At sites of atherosclerosis, for instance, endothelial cells activate and stimulate the expression of survival effectors as well as pro-apoptotic agents. Endothelial cells also activate the expression of adhesive molecules. Differential expression of at least one the protein may be used to monitor the expression of these genes and to validate ex vivo atherosclerotic phenotypes in cell based screening models under conditions that stimulate vascular injury. This may include HUVEC and BAEC as well as permanent cell line exhibiting endothelial cell phenotype. Cultured monolayers can also be exposed to fluctuating shear stress in specialized apparatus.
- **Detection of mRNA :** To detect differentially expressed proteins and associated genes, mRNA can be isolated and amplified from tissue section, cell extract or biopsies, using routine protocols in the art. Transcript within the RNA sample may be detected by utilizing hybridization technologies such as DNA chip technology containing specific probe sequences or RT PCR using specific oligonucleotides that are specifically designed to monitor the differential expression of the gene. Expression can then be corroborated with routine technologies including quantitative RT-PCR or northern blot analysis.
- **Detection of protein :** The presence of at least one of the protein can be detected in atherosclerotic tissues by routine immuno-histochemistry. The protein can also be detected via an ELISA assay or utilizing mass spectroscopic technologies following protein isolation in a two dimensional gel eclectrophoresis apparatus. The two hybrid system may also be used to detect intracellular proteins that may associate with at least one of the protein during the development of an arterial plaque and the formation of foam cells.

### 2) Inhibitors for controlling differential expression of at least one of the protein during atherosclerotic plaque growth and erosion

Methods that can be used for the identification of agents controlling the expression and activity of at least one protein of the group in a growing arterial plaque are multiple.
- **Cell based assays :** One of the protein may be used to identify molecular entities that modulate the formation of a foam cells using macrophages or permanent cell lines based screening assays in conditions that reproduce the development of an atherosclerotic plaque. This may include but not limited to ,THP1 cells (ATCC # TIB-202, U937 cells (ATTCC # CRL1593). Monocyte/macrophages may be isolated using routine protocols and stimulated with but not limited to, oxLDL and components from hyperlipidemic serum. Either one of the molecules, may also be used in a screening assay for the identification of agents that can protect against endothelium dysfunction. Sources of endothelial cells may be, but not limited to, HUVEC or BAEC.
   These cell based assays may be phenotyped as atherosclerotic cells, using differential expression of at least one protein expression in association with the expression of atherosclerosis associated genes and used to detect novel associated genes.
   These cell based assays may also be used to screen for compounds that are capable of controlling the expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, and/or corresponding protein, and limiting the growth and instability of an atherosclerotic lesion. Thus, cell based assays using the detection of differential expression of the above genes may be used to identify drugs , pharmaceuticals, therapies, and interventions which may be effective in treating arterial plaque growth and rupture.
- **Animal based systems :** Animal based systems may include genetically modified or not modified animals. Recombinant animal models may include, but is not limited to, ApoE and ApoB deficient mice, ApoR deficient pigs. Non recombinant animal model may include rabbit, rat , mouse and pigs. The expression of at least one of the protein in these animal models may be used for phenotyping, and strain selection for atherosclerotic diseases.
   The example presented hereafter demonstrates the generation, phenotypic characterization and usefulness of pig expressing at least one the following protein in an early atherosclerotic lesion. Differential expression in these animals may be used for screening, validation and optimisation of drug candidates.

### 3) Assays for compounds that interfere with interaction of at least one of the protein and other cellular compounds

Differential expression may in vivo interact with one or more intracellular compounds within an atherosclerotic tissue. Those compounds may include intracellular proteins, phospholipids, fatty acids, and small molecules. Agents that can interfere with these interactions may be useful in regulating foam cell formation and plaque growth and stability. Any assay system which will allow interaction of at least one of the protein and cellular compounds under circumstances that mimic the development of an atherosclerotic lesion or from an atherosclerotic plaque versus vascular cells from non atherosclerotic vessel wall, will be convenient. Alternatively, arrays that can rise at least one protein or different protein in combination may be used to screen for molecules that can interact with at least one of said protein. Therefore, protein arrays will be convenient. The formation and the inhibition of the complex can be quantitatively or qualitatively detected using fluorescent labelling. The reaction can be conducted in a solid phase assay or in a liquid phase. Antibodies can be used as a signal amplifier either in the liquid phase or in the solid phase.

### 4) Monitoring of effects during clinical trials

Monitoring the effect of a drug candidate for treating atherosclerotic growth and plaque instability using tools to detect differential expression at least one of the protein may be applied in clinical trials. For example, differential expression may be used to study drug efficacy in human tissue section by immunohistochemistry or in situ hybidization. Expression may be or not associated with plaque imaging and be used for monitoring patients at risk.

### 5) Antibodies with potential therapeutic activity in atherosclerosis

Antibodies that modulate differential expression of at least one of the protein in arterial lesions and can interfere with the cellular activity of the protein in an atherosclerotic plaque, may be used for controlling plaque growth and stability. Such antibodies include polyclonal antibodies, murine and human monoclonal antibodies, single chain antibodies, Fab fragments and chimeric antibodies.

### 6) Imaging atherosclerotic plaque

As shown in the present invention, at least one of the protein is up regulated in the vascular wall at sites that are prone to develop an atherosclerotic lesion. Diefferential expression of at least one of the protein may thus be used for non invasive imaging of the growth, erosion and stability of an arterial plaque at sites of ischemia. As described in the example hereafter Stearoyl CoA desasturase, Aldose reductase and aldehyde reductase, sphingomyelinase, acid ceramidase, Ceramide glucosyl transferase, sphingosin phosphate liase, thymosine beta 4, aldehyde dehydrogenase, ATP ase Ca++ binding protein and CD163 are up regulated in a plaque and can be used to label endothelial cells or foam cells within the plaque. This may constitute an excellent tool for monitoring the development and /or the regression of the plaque and to develop an appropriate therapeutic strategy.

Non invasive imaging can be performed with different marker including monoclonal antibodies labelled with radioisotopes or specific ligand that can be designed based on the structural parameters of stearoyl CoA desaturase.

### EXAMPLES

The following examples are offered to illustrate the invention, but not to limit the present invention.

### Example 1 : Animal model and sample preparation

Differential gene expression analysis during the progression of an atherosclerotic plaque may be applied to a variety of animal models for the detection of co regulated pathways that may constitute targets implicated in the growth and the erosion of atherosclerotic lesions. These animals may be used for screening or validation of molecules that can modulate the differential expression of at least one of the protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163 at the level of an arterial plaque. Animal based systems may include non genetic and genetic modified animals such as, but not limited, pigs, mouse, rat, rabbit, ApoE negative mouse, ApoB negative mice and ApoR mutant pig.

In the present invention, a mini pig model was used to monitor the differential expression of genes during the development of an atherosclerotic plaque under dietary supplementation using a cholesterol rich diet.

These mini pigs were obtained by crossbreeding Gottinger and Yucatan minipigs (Charles-River laboratories). They were housed in a temperature-controlled room (to 20±1°C) at 50±2% humidity on a 12-hour/12-hour light/dark cycle. The investigation conforms the *Guide for the Care and Use of Laboratory Animals* published by the US National Institutes of Health (NIH Publication No. 85-23, revised 1996). All experimental procedures for these animals were performed in accordance with protocols approved by the Institutional Animal Care and Research Advisory Committee.

Atherosclerosis was induced by feeding the animals a diet containing 4% cholesterol, 14% beef tallow, and 1% hog bile extract in daily amounts of 1000 g. Water is provided ad libidum.

### 1) Cardiac Catheterization

Immediately prior to sacrifice the animals were sedated with 1 mL azaperone IM (Stresnil 40 mg/mL, Janssen Pharmaceutica) and premedicated with 7 mg/kg ketamine IM (Imalgene 100 mg/mL, Janssen). The animals were incubated and artificially ventilated with a mixture of 30% oxygen and 70% room air (Mark 7A Bird respirator). Arterial blood gases were checked at regular intervals and the ventilation adjusted to maintain normal blood gas values. Anesthesia was maintained by a continuous intravenous infusion of sodium pentobarbital (Nembutal 60 mg/mL, Signify) at a rate of 3 mg · kg⁻¹ · h⁻¹. Arterial access was achieved by surgical isolation and cannulation of the left carotid artery. The animals were then given 200 IU/kg heparin and 1 mg/kg IV of 2% lidocaine (Xylocaine 20 mg/mL, Astra) before manipulation of the coronary arteries.

### 2) LAD sections

Briefly, Lad was perfused with cold NaCl 0.9% via aortic root, carefully dissected and cut into 7-µm sections.

For gene expression studies, LAD was embeded in OCT and snap frozen in liquid nitrogen until sectioning. For histological and immunohistological analysis, LAD was transferred to embedding cassette in methanol 70% till paraffin embedding

### 3) Histomorphometric and Immunohisto chemical Analysis

Seven µm sections of the proximal LAD were stained with hematoxylline-eosine to assess lesion size. Morphometric analysis of sections was performed using the Leica Quantimet 600 image analysis system (Leica, Brussels, Belgium). The external elastic lamina area (EEL), internal elastic lamina area (IEL), medial, intimal, and luminal areas are measured. Total lipid deposition in the lesions was determined using oil-red-O staining. The total amount of collagen in the lesion was determined on picrosirius red stained sections viewed in normal light. Triple helix collagen was measured on the same sections viewed in polarized light. Elastin content was measured on Verhoeffs-stained sections and by measuring autofluoresence of the coronary lesion. Atherosclerotic lesions were classified using the Stary classification into early lesions and more advanced lesions.

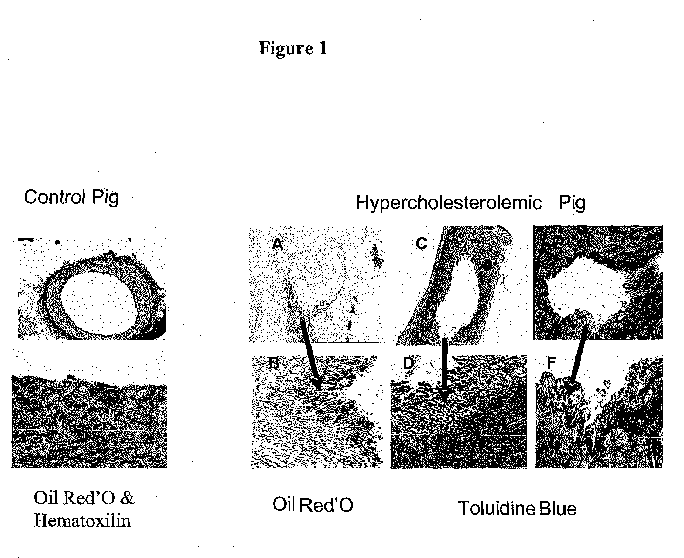

Examples of arterial cross sections showing early and advanced plaques containing lipids and macrophages are illustrated in figure 1.

### 4) Seric lipids and glucose measurement

Peripheral venous blood was drawn from an ear vein. Total cholesterol, HDL cholesterol and triglyceride levels were measured by enzymatic methods (Boehringer Mannheim, France). LDL cholesterol levels were calculated with the Friedewald formula. Plasma oxidized LDL (ox-LDL) was measured with a mAb-4E6 based competition ELISA. The monoclonal antibody is directed against a conformational epitope in the apoB-100 moiety of LDL that is generated as a consequence of substitution of lysine residues of apoB-100 with aldehyde residues. The C50 values, i.e. concentrations that are required to obtain 50% inhibition of antibody binding in the ELISA, are 25 mg/dL for native LDL, and 0.025 mg/dL for oxidized LDL with at least 60 aldehyde-substituted lysines per apoB-100.

Plasma levels of C-Reactive Protein (CRP) was measured with an immuno turbidimetric assay (Roche) with a detection limit of 3mg/l.

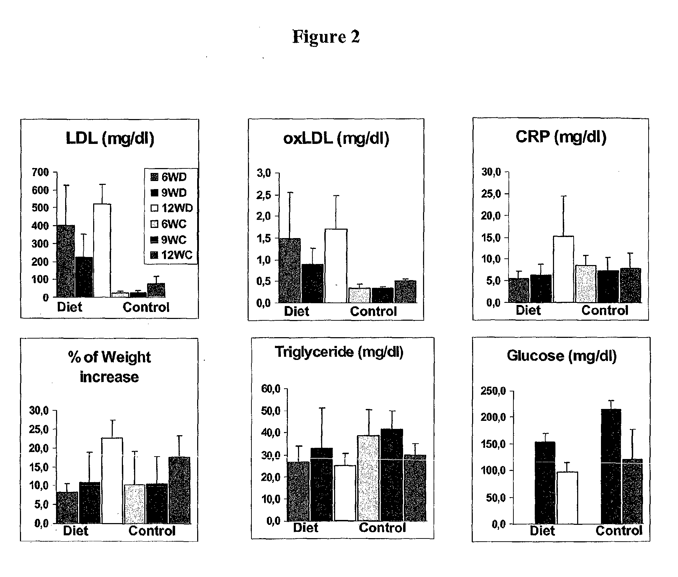

Figure 2 illustrates the different parameters of this pig model, indicating that this animal model is a true hypercholesterolemic model with absence of hyperglycemia and hypertriglycerimia.

### 5) Monocytes isolation

Blood was drawn into 4% sodium citrate and centrifuge 10 min, washed two times in HBSS at 3120 g (4500rpm), 10 min at 20°C. Leukocyte isolation was performed by a ficoll -Histopaque Gradient (1.119) as described by the provider (Sigma, ) and monocytes were isolated using CD14 magnetic microbeads (Miltenyi ). Cells were washed twice, lysed with trizol reagent and stored at -80°C.

### 6) Plaque extraction and mRNA amplification

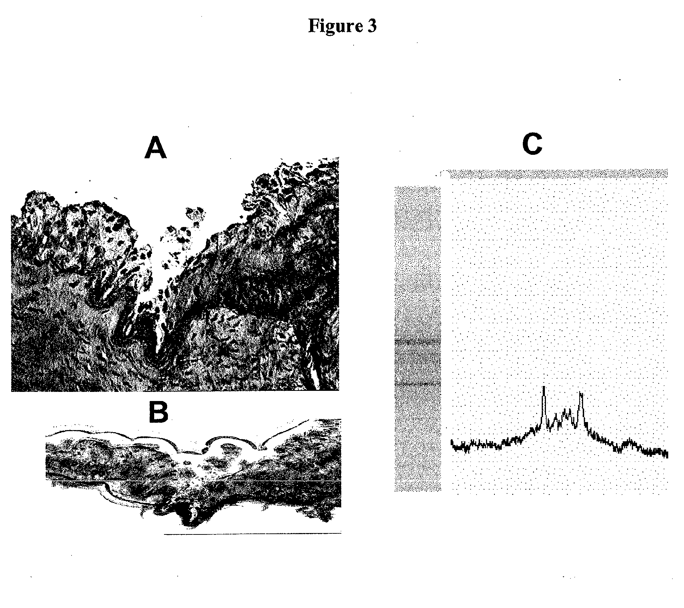

To be physiologically relevant and to be associated with the progression of a plaque, differential gene expression must be quantitatively detected at the level of cells that are recruited during the growth of an atherosclerotic plaque. This can be monitored with microdissection technologies. In the present invention, the following method was used:
- **Laser capture micro-dissection (LCM) :** LAD were sectioned at 8 µm in a cryostat, mounted on polylysine coated glass slides (two sections per slides). The slides then were stored at - 80°C.
   For lesions phenotyping, one every 20 twenty slides was stained by Oil Red O (slides were dipped just before defrosting in ORO solution (72 mg ORO, 24ml isopropanol, 16 ml RNase-free water) for 10 minutes and rinse in two bathes of H2O). The following slides were stained by Toluidine blue (slides were dipped just before defrosting in 75%EtOH for 4 sec, stained in a bath of Toluidine blue (dissolved at 0.1%w/v in PBS) solution for 8 sec, rinse in RNase-free water, deshydrated in 75% ethanol for 30sec).
   Before microdissection, the frozen sections were fixed in 75% ethanol for 30 sec, rinsed in RNase free water in order to remove the OCT, dehydrated for 30sec in 75%, 95% and 100% ethanol and 3 min in xylene successively. Once air-dried, the tissues were laser-capture microdissected by a PixCell II LCM system using Capsure HS LCM caps following the manufacturer's protocols (Arcturus Engineering, Mountain View, CA).
   A typical plaque capture experiment is illustrated in figure 3.
- **RNA extraction :** Total RNAs were extracted from either circulating monocytes or laser-capture cells from one entire LAD section with the RNeasy Mini Kit (Qiagen) according to the manufacter's recommendations.
   Total RNA from monocytes were extracted using Trizol solution (In vitrogen) and PLGI-Heavy Phase Lock gel (Eppendorf).
   Optical density was measured for each sample with a biophotometer (Eppendorf) using disposable cuvettes.
   Quality of the tRNA preparation from monocytes and from one entire section of LAD was visualized with the eukaryote total RNA nano assay using the Agilent 2100 Bianalyzer (following the manufacturer's protocols).
   Figure 3 exemplifies the quality of the capture and of the RNA extract.
- **cDNA synthesis :** All purified RNA from microdissected cells or 500ng to 5µg of monocytes tRNA was mixed with 1µl of 10mM dNTP mix and 1µl of 20mM T7-(dT)24 primer in 10µl final volume, incubated for 5 minutes at 65°C and chilled on ice. Next, 4 ul of 5X First-strand reaction Buffer, 2µl of 0.1 M DTT and 1µl RNaseOUT Recombinant Rnase Inhibitor (40U/ul) were added and placed at 42°C for two minutes, 200U of Superscript II RNase H⁻ RT (In vitrogen) were added and the reaction kept at 42°C for 1 hour. Next, 30µl 5x second strand reaction buffer, 10 mM dNTP mix (3µl), 4µl DNA polymerase I (10U/µl), 1µl E.Coli DNA ligase (10U/µl), 1µl RNase H (2U/µl) and 91 µl of RNase-free water were added and the reaction mixture was incubated at 16°C for 2h, followed by incubation of 10min at 16°C after addition of 2µl of T4 DNA polymerase (5U/µl). The reaction was stopped by adding 10µl of EDTA 0.5M. Next, the cDNA was extracted with phenol-chloroform-isoamyl alcohol using PLGI-light Phase Lock gel, and precipitated with NH4OAc and ethanol in presence of 5µg of glycogen.
- **T7 RNA polymerase amplification (aRNA) :** The MEGAscriptTM T7 kit (Ambion) was used : 8µl double-stranded cDNA, 2µl Ambion transcription buffer, 2µl each of 150mM ATP, CTP, GTP and UTP and 2µl Ambion T7 Enzyme mix were mixed and incubated at 37°C for 6 hours. Next, aRNA were extracted with phenol-chloroform-isoamyl alcohol using PLGI-Heavy Phase Lock gel and cleaned up using RNeasy Mini Kit. The volume was reduced in a speed vac.
- **Second round of aRNA amplification :** First, aRNA (from first round amplification was mixed with 250ng random hexamer and 1µl of 10mM dNTP mix, incubated at 65°C for 5 minutes and then chilled on ice. Next, 4 ul of 5X First-strand reaction Buffer, 2µl of 0.1 M DTT and 1µl RNaseOUT Recombinant Rnase Inhibitor (40U/ul) were added. The reaction was left to equilibrate at room temperature before to add 200U of Superscript II RNase H⁻ RT (In vitrogen), the reaction was then incubated first at room temperature for 10 min then at 42°C for 50 min. Then, 1µl of RNase H was added and the reaction incubated at 37°C for 20 min, after which the reaction was heated to 95°C for 2 min and chilled on ice. For second strand cDNA synthesis, 2 µl of 20 µM T7-(dT)24 primer were added and the mixture incubated at 70°C for 5 min and 42°C for 10 min.. Next, 30µl 5x second strand reaction buffer, 10 mM dNTP mix (3µl), 4µl E. Coli DNA polymerase I (10U/µl), 1µl RNase H (2U/µl) and 89 µl of RNase-free water were added and the reaction mixture was incubated at 16°C for 2h. Then, 2µl of T4 DNA polymerase (5U/µl) were added and the reaction incubated at 16°C for 10 more min before to be stopped by the addition of 10µl of 0.5M EDTA. The double stranded cDNA was extracted with phenol- chloroform-isoamyl alcohol using PLGI-light Phase Lock gel to get rid of proteins, and precipitated with NH4OAc and ethanol in presence of 5µg of glycogen. The cDNA was then resuspended in 8µl RNAse-free water and use for second-round T7 in vitro transcription as above except that the incubation last only three hours at 37°C.

After phenol-chloroform-isoamyl alcohol and RNeasy Mini Kit cleanup of the aRNA, the density optic was measured and the concentration and the size distribution of aRNA was analysed with the mRNA smear nano assay using the Agilent 2100 bioanalyzer (following the manufacturer's protocols).

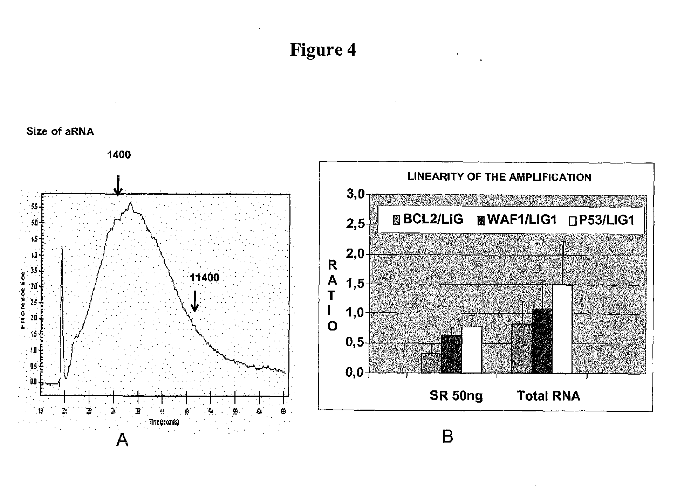

Following a second run of amplification the aRNA sample was tested for quality. This included, size distribution and preservation of the relative abundance of the RNA. Figure 4 illustrates these quality controls. The relative abundance of aRNA was certified using low, medium and high activity gene markers.

### Example 2 : Differential expression

Differential expression of genes in a given sample, can be monitored with different technologies including, traditional northern blot, RT-PCR, and differential display. However, methods and assays of the invention are most efficiently designed with array and DNA-chip technologies.

Any hybridization format may be used, including solution based and solid support based formats. In the present example , a high density array of DNA probes on a solid support was preferred with the following protocol.

### 1) Preparation of Pig Universal Reference

A pig universal reference was made. Total RNA was extracted with Qiagen RNeasy (Qiagen) from 8 swine control organs, including the heart, brain, lung, liver, kidney, spleen, thymus, and aorta. Total RNA from each organ was amplified as indicated before for microdissected samples. Finally, the Pig Universal Reference was made by equimolar mix of aRNA (first round and second round) of 8 swine control organs.

### 2) Preparation of the labeled pig cDNA sample for DNA chips analysis

Fluorescently-labeled cDNA was prepared and purified according to an Agilent protocol (Agilent Direct-Label cDNA Synthesis Kit Protocol, Agilent, Palo Alto Ca). 4 µg of swine aRNA and 2.5µg of random hexamer (In vitrogen) were used per reverse transcription reaction. Cy3- and Cy-5 dCTP (NEN Perkin Elmer) was incorporated into cDNA during reverse transcription. For purification with QIAquick PCR Purification Kit (Qiagen), three washes with buffer PE were performed. Paired cDNA were dryed under vacuum in a rotary dessicator.

### 3) Preparation of the hybridization mixture and Hybridization

Agilent Human cDNA Microarrays ( Agilent, Palo Alto, CA) were hybridized according to Agilent supplier instructions with minor modifications. Cyanine 3-/cyanine 5 labeled cDNA sample was resuspended in 5.96µl of nuclease-free water and the following mix was added per sample :
- 1.26µl Deposition Control Targets (sp300 operon, Qiagen)
- 2.28 Cot-1 DNA (InVitrogen)
- 9.5µl 2x Deposition Hybridization Buffer

After incubation at 98°C for two minutes to denature the cDNA, and centrifugation at 10,000g (13000rpm) for 5 minutes, 16µl on 19µl of hybridization mixture were transferred in a new amber tube to eliminate the pellet. Finally 12 µl were applied to the microarray under a 24/30mm coverslip (Corning) for 17h at 60°C in a waterbath, in Scienion hybridization chamber.

Each pig labelled sample was combined with the labelled Pig Universal Reference and the dye swap combined cy3/cy5 samples were hybridized on the two arrays on the same slide.

Washes were performed as recommended by Agilent supplier except that wash 1 was performed during 30 minutes twice and wash 2 was performed during 12 minutes twice. Finally slides were dried by centrifugation 10 minutes at 400g at room temperature.

Slides were scanned with an Agilent scanner (Agilent G2565AA Microarray Scanner System), with a resolution of 5 microns. Signal extraction was performed with Feature Extraction version 5 (Agilent G2566AA Feature Extraction Software; Agilent Palo Alto, CA). Output files were XML and Text file and visual results. Configuration parameters were the following :
- In the general configuration, "Spot finder", "PolyOutlierFlagger" and "CookieCutter" were selected.
- In Find Spots configuration, "Autofind corners" was selected with a "Dev Limit" of 70 microns.

In CookieCutter configuration, "Reject based on IQR" of 1.42 for Feature and Background was selected.
- In the PolyOutlierFlagger configuration, "Non-Uniformity Outlier Flagging" and "Population Outlier Flagging" were selected with the default parameters.

### 4) Gene expression analysis

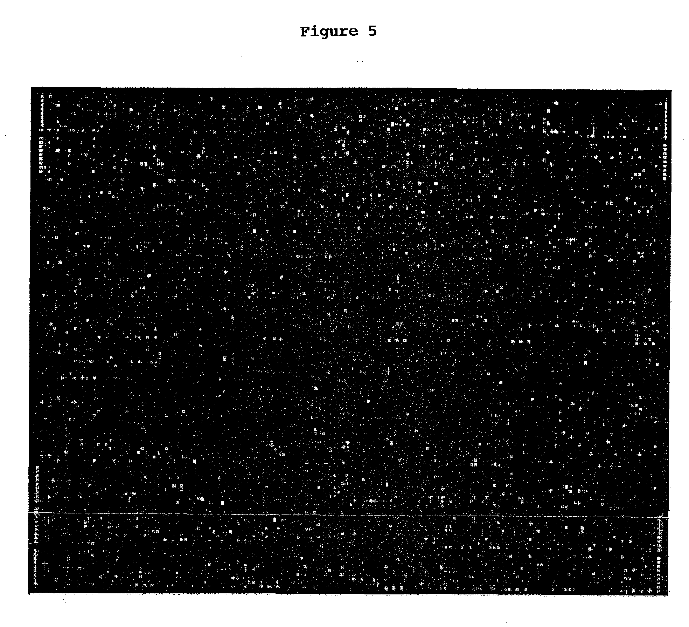

The gene expression patterns were individually determined for each sample. In a typical experiment, samples of three control pigs and four diet supplemented pigs were analysed in reference to the universal pig signature after 6, 9, 12, and 24 weeks of diet. Control samples were either circulating non activated monocytes from non diet pigs or laser captured endothelial cells from non diet pigs. Figure 5 illustrates a typical expression signature, and table 1 hereunder indicates the set of genes that were reproducibly up regulated in an atherosclerotic plaque.

**Table 1**

| Reference Genes | Fold Expression Mean value | Novel Genes that are differentially expressed In an Atherosclerotic plaque | Fold Expression Mean value |
|---|---|---|---|
| ABCA1 | 3.0 | Stearoyl CoA Desaturase | 3.2 |
| CD68 | 2.1 | Aldehyde dehydrogenase | 1.9 |
| CD36 | 4.7 | Aldehyde Reductase AKR1 A1 | 2.0 |
| LDL - R | 2.0 | Aldose Reductase AKR1 B1 | 2.0 |
| PECAM | 2.0 | Thymosine beta 4 | 5 |
| HSP60 | 2.3 | Sphingomyelinase | 2.3 |
| HSP70 | 2.4 | Sphyngosine Phosphate liase | 3 |
| TLR4 | 1.9 | Acide Ceramidase | 2.1 |
| Erg 2 | 2.6 | UDP-glucose ceramide glucosyl tranferase | 2.2 |
| VEGF | 1.9 | ATPase Ca++ transport binding protein 1 | 6.5 |
| Paraoxanase3 | 1.4 | CD 163 | 3.05 |
| BRCA1 | 4.2 | | |
| HIF1 | 2.3 | | |
| IL1 - R | 1.5 | | |
| ATF3 | 1.6 | | |
| NADH dehydrogenae | 2.1 | | |
| HCTGF | 2.4 | | |
| Galectine 3 | 3.0 | | |

These genes which are named positive genes, were co-expressed with a set of canonical genes which are listed in table 1 hereunder. Table 1 indicates a combination of canonical genes and novel genes that were not described before to be up regulated during the progression of an atherosclerotic plaque. This set of genes represents a novel gene signature for atherosclerosis and identifies different metabolic pathways that are positively regulated during the pathogenic process and contributes to the drastic changes in expression at the level of a vascular lesion where an atherosclerotic plaque can develop.

### Example 3 : Novel genes associated with the progression of an atherosclerotic plaque

### 1) Stearoyl CoA desaturase

Stearoyl CoA desaturase is differentially expressed at the cellular level in early lesions, containing activated endothelial cells and macrophages, together with genes that are known to be involved in the process of atherosclerosis,

The exact mechanism by which stearoyl CoA desaturase may influence plaque growth and instability is unknown.

Stearoyl CoA deasaturase is a member of a family of genes that are regulated by sterol regulatory element - binding proteins (SREBPs). This includes, acetyl CoA carboxylase (ACC), fatty acid synthase (FAS), glycerol 3 - phosphate acetyltransferase (GPAT) and Delta 6 and Delat 5 desaturases.

Stearoyl CoA desaturase is the rate limiting enzyme in the biosynthesis of monosaturated fatty acids. It catalyzes the formation of palmitoleate (delta 9, 16:1) and oleate (delta 9, 18:1) from palmitate (16:0) and stearate (18:0) which are the major constituent of membrane phospholipids and triacylglycerol stores found in adipocytes (Kasturi R and Joshi V.C., 1982, JBC, 257, 12224-12230 ; Ntambi J.M., 1995, Prog. Lipid Res., 34, 139-150).

Stearoyl CoA desatuase has been shown to play a role in lipogenesis and in adipocyte differentiation. Gene expression is elevated in liver tissue and adipose tissue and has been shown to control the serum level of triglycerides and fatty acids (Jones B.H. et al 1996, Am J. Physiol., 272, E44-E49; Pan D.A. et al 1994 , J. Nutr. 124, 1555-1565 ).

The role of Stearoyl CoA desaturase in hepatocyte triacylglycerol metabolism and in adipocyte differentiation is well documented. Transcriptional up regulation is induced by dietary factors, metals, peroxisomal proliferators, hormone such as insulin and metabolites such glucose (Park E.I., et al 1997, J. Nutr. 127, 566-573;/ Casimir D.A., & Ntambi, J.M., 1996, J.B.C., 271, 29847-29853;/ Ntambi J.M. et al 1996, Biochem. Biophys. Res. Com. 220, 990-995 ). Down regulation is observed in the presence of polyunsaturated fatty acids and during adipose tissue differentiation. Thus, Stearoyl CoA desaturase exhibits target characteristic for the treatment of obesity. Targeted disruption of the gene in a mouse model revealed that the enzyme plays a direct role in the biosynthesis of cholesterol ester, triglyceride and wax ester. Stearoyl CoA desaturase deficient animals, are deficient in hepatic cholesterol and triglycerides. The mice are leaner than normal and exhibit defects in lipid metabolism (Miyazaki M et al, 2001, J. Nutr. 131, 2260-2268 ; Ntambi, J.M. et al 2002, PNAS, 99, 11482 -11486).

The present invention is based on the unexpected discovery that beside the hepatic and the adipocyte tissues, Stearoyl CoA deasturase is differentially expressed in tissues that are constitutive of an early atherosclerotic plaque, in hyperlipidemic conditions that are relevant with the development of atherosclerosis, and is coexpressed with known atherosclerosis associated genes in the same injured tissue. This allows the identification of a target in pathways that are useful for identification of agents with both diagnostic and therapeutic activity in atherosclerosis.

Mechanisms that control transcriptional up regulation of the Stearoyl CoA desaturase gene during the growth of an atherosclerotic plaque are unknown. Using permanent cell lines, it was shown that expression of Stearoyl CoA desaturase is negatively regulated by PPAR gamma agonists such as thiazolidinediones during adipocyte differentiation ( Kim Y-C et al , 2000, J. Lipid Res. 41, 1310-1316 ). This contrasts with known effects of PPAR gamma agonists on atherosclerosis associated genes expression during macrophage differentiation and foam cells formation in an arterial plaque. Thus Stearoyl CoA desaturase may have different effects in liver cells, vascular cells and adipocytes and may exert a specific role in the development and the stability of an atherosclerotic plaque.

Based on this unexpected up regulation in the atherosclerotic lesion, the present invention provides methods to monitor the differential expression of Stearoyl CoA desaturase for diagnostic and prognostic purpose and to identify compounds that are capable to increase or decrease the activity of Stearoyl CoA desaturase to specifically reduce the size of a plaque, its erosion and to stabilize the plaque.

### 2) Aldehydes Reductase : Aldo Keto Reductase family 1 member B1: AKR1B1 (EC 1.1.1. 21) ; Aldo Keto Reductase family 1 member A1: AKR1A1 (EC 1.1.1.2)

AKR1A1 and AKR1B1 are members of the aldo ketose reductase super family which includes a number of related monomeric NADPH-dependent oxidoreductases such as aldose reductase, xylose reductase, prostaglandin F reductase, and many others ( Jez J.M. et al 1997 Biochemical Pharmacology 54, 639-647 ). The enzymes are closely related monomeric proteins but exhibit different substrate specificity. AKR1B1 is a low Km aldose reductase enzyme and is involved in the polyol pathway. The enzyme controls the reduction of aldose such as glucose and galactose to their corresponding polyol such as sorbitol and galactilol.

This enzyme controls the level of glucose in the blood and exhibits the characteristics of a pharmacological target for treating diabetes and its hyperglycaemic complications such as, neuropathy, retinopathy, nephropathy, and micro angiopathy (Mylari B,J, US 20020143017).

Its precise function in the pathogenesis of atherosclerosis and more specifically, in the progression of a plaque is totally unknown.

AKR1A1 is a high Km aldose reductase. At elevated blood glucose levels, a significant flux of glucose through the polyol pathway is induced in tissues like nerves, retina, lens and kidney. Activation of the polyol pathway is therefore considered to induce diabetic complications. Aldose reductase inhibitors are used to prevent or reduce these complications. These inhibitors however, demonstrate an imperfect control of blood glucose and their beneficial effects are far satisfactory. Two main classes of orally active aldose reductase inhibitors have been reported, with Sorbinil and Tolrestat being the most representative members of each family. The in vivo activities of these two products are very different and some of them have been shown to cause liver complications and hypersensitivity reactions when used to control glucose production in diabetes patients (Costantino L, et al 1997, Exp. Opin. Ther. Pat. 7, 843 - 851). For these reasons, the search for new molecules with better pharmacological properties against these reductase is an active area.

In addition to their implication in the polyol pathways, AKR1 A1 and AKR1 B1, express an aldehyde reductase activity with different substrate specificity. Both enzyme are implicated in the glycerolipid pathway and catalyze the reduction of lipid derived aldehyde to generate glycerol. Glycerol is involved in the biosynthesis of triacylglycerol via the production of glycerol 3 phosphate and the metabolism of glycerolipids. Therefore, activation of the AKR1 A1 and/ or AKR1 B1 aldehyde reductase activity in macrophages during the development of the atherosclerotic plaque may be responsible for an over expression of triacylglycerol and the accumulation of foam cells at the level of the plaque.

Oxidation of circulating Low Density Lipoprotein (LDL) and their uptake by macrophages via scavenger receptors, is the major reaction that promotes the recruitment and accumulation of lipid-laden macrophages in the vessel wall, leading to fatty streaks that precede the development of a plaque. Lipid peroxidation which occurs in these foam cells during atherosclerosis, generates high concentration of breakdown products which may be toxic or mitogenic to other vascular cells and may be responsible for the progression of the plaque. Among these down products, aldhedydes are the end products of lipid peroxidation and exhibits high reactivity with different biomolecules that may be implicated in the pathogenesis of atherosclerosis. Unsaturated aldehydes for instances, are derived from the oxidation of poly unsaturated fatty acids such as linolenic and linoleic acids which are particularly abundant in oxidized LDL (Morisaki N. et al , 1985, J. Lip. Res. 26, 930- 939).

Therefore, in addition to being responsible for the production and accumulation of high amount of active glycerol and triacyl glyceryl in foam cells, activation of the aldehyde reductase activity may generate intracellular or secreted active down products that may activate the atherosclerotic process. The exact mechanisms by which these aldehydes regulate the growth, the stability or the regression of an atherosclerotic plaque are totally unknown. Reactions other than the polyol pathway may be activated by the AKR1 family.

AKR1 A1 and AKR1 B1 have different substrate specificity. In addition, major differences exist in the function and tissue specific expression of aldehyde reductase and aldose reductase (O'Connor T et al, 1999, Biochem J. 343, 487-504). It was recently shown for instance, that perfusion induced ischemia influences aldose keto reductase but not aldehyde reductase activity in heart, and that specific aldose keto reductase inhibitors were cardioprotective. The activation of aldose reductase activity in ischemic heart was not due to increased expression but to activation of the enzyme by endogenous factors ( Hwang, Y.C., December 2001, FASEB J., 10. 1096 ). Thus aldose reductase and aldehyde reductase activities clearly express different tissue specific functions and are clearly involved in different pathways. AKR1 A1 preferentially catalyzes the NADH dependent reduction of aliphatic aldehydes, aromatic aldehydes, and biogenic amines. While AKR1 B1 expresses also an aldehyde reductase activity, the enzyme better catalyzes the NADH dependent reduction of aldopentoses, aldohexoses. Therefore, while both enzymes catalyses the reduction of lipid derived aldehydes, AKR1 A1 appears to be a better enzyme for aldehyde substrates. Using molecular docking and data base screening, it was recently shown that new series of inhibitors with a better specificity to the aldose reductase AKR1 B1 when compared to aldehyde reductase activity of AKR1 A1 could be designed , suggesting that the reverse strategy might be possible ( Rastelli G. et al 2002, Bioorganic & Medicinal Chemistry 10, 1437-1450 )

The present invention demonstrates for the first time that these enzymes are up regulated at the transcription level in early and advanced atherosclerotic plaques under conditions of hypercholesterolemia and in the absence of high level of blood glucose and insulin. This suggest that these enzymes may have a specific implication in the macrophage dependent lipid metabolism.

Therefore, the present invention, relates to compounds and methods using the differential expression of an aldehyde or aldose reductase activities in an atherosclerotic plaque relative to their normal expression to discover new products that specifically reduce this reductase activity, to prevent or control the production of lipid dependent aldehyde derived down products.

Specifically, the present invention identifies ways to treat patients with atherosclerosis in the absence of increased levels of circulating triacyl glycerol and glucose thus allowing treatment of atherosclerosis in the absence of hyperglycemia and avoiding potential metabolic side effects of drugs that lower sorbitol and are normally used for the treatment of hyperglycemia. This invention relates to pharmaceutical compositions that contain a specific aldehyde reductase inhibitor and to methods using such compositions to treat or prevent the accumulation of foam cells, the progression and the instability of an atherosclerotic plaque in mammals under hypercholesterolemic conditions.

### 3) Aldehyde dehydrogenase, ALDH1 (EC 1.2.1.3)

Aldehyde dehydrogenase is one of the major enzyme of the alcohol metabolism , next to the alcohol dehydrogenase ( ADH 103700 ). The protein belongs to the NAD-dependent aldehyde dehydrogenase family which contains ALDH I, II, III, and IV encompassing over twenty different isoforms.

The catalytic role of ALDH is well known. ALDH is the enzyme that catalyzes the hydrolysis of esters as well as oxidize aldehydes into acids. The enzyme has been found to be involved in different metabolic pathways, including the fatty acid pathway, bile acid biosynthesis, glycerolipid metabolism, tryptophan metabolism, among others.

An inactive dominant mutant form of ALDH1 was described in 1979 in Asian populations (Goedde et al Hum Genet. 51, 331-334). Loss of enzymatic activity in these individuals was the result of a point mutation (Yoshida et al, 1984, Proc. Natl. Ac. Sci. USA 81, 258-261 ). Interestingly enough, this inactive mutant did not display any metabolic abnormalities. Therefore, this molecule appears to be an excellent target for the design of small molecules to control its activity in patients.

For a long time, this enzyme was considered as a target for the treatment of patients with alcohol sensitivity and for the treatment of alcoholism and alcohol abuse. The present invention describes for the first time, a positive differential expression of this enzyme in an atherosclerotis plaque.

The role of ALDH1 in the development of atherosclerosis is totally unknown.

ALDH1 is cytosolic, exhibit a high Km for acetaldehyde and has been assigned a major role in glyceraldehydes detoxification. The enzyme has two distinct catalytic activities and exhibit both esterase and dehydrogenase activities (Duncan R J; 1983 Biochem .J. 230, 261-267 and Tu GC and Weiner H. 1988, J. Biol. Chem., 263, 1218-1222). The existence of specific inhibitors of the esterase and the dehydrogenase activities has been demonstrated, (Abriola and Pietruszko, 1992, J. Protein Chem., 11, 59-70). Accumulation of acetaldehyde in blood is observed when ethanol is ingested and is accompanied by marked increases in heart rate and cardiac output as well as by decreases of vascular resistance. These changes were reversed by inhibiting ALDH1 activity (Kupari et al 1983, Alcohol Clin Exp Res 7, 283-288).

Alternatively, ALDH1 is also involved in the fatty acid metabolism pathway and is reported to generate aldehyde derivatives from fatty acid. Thus the presence of ALDH1 in a growing atherosclerotic plaque, may be responsible for the production and accumulation of cytotoxic aldehyde derivatives.

### 4) Thymosin β 4

Thymosin β 4 is a member of the Thymosin super-family which comprises highly conserved polar polypeptides ranging in molecular weight from 1 to 15 kDa, and originally thought to be thymic hormones. In 1990, Thymosin β 4 was identified as an intracellular G actin sequestering peptide (Safer D., and Golla V.T., 1990, PNAS, 87, 2536-2540).

Thymosin β 4 has been reported to have an effect on the differentiation of T lymphocytes (Low, T.L.K. et al , 1981, PNAS, 78, 1162-1166 ), and to inhibit the migration of macrophages ( Weller F.E., et al, 1988, J.Biol. Resp. Modif. 7, 91-96). More recently, Thymosin β 4 has been shown to stimulate endothelial cells attachment and spreading and to increase the production of matrix metalloproteinases that may degrade the basement membrane ( Grant D.S. et al, 1995, J.Cell Sci. 108, 3685-3694 , Malinda K.M. et al 1997, FASEB J. 11, 474-481 ). It was finally shown that Thymosin β 4 sulfoxide can be produced by monocyte and act as an anti-inflammatory agent ( Young J.O. et al, 1999, Nat. Med. 5, 1424-1427 ).

The exact mechanism by which Thymosin β 4 influences cell migration and spreading was established in 1991 (Safer D., et al J.B.C. 266, 4029-4032). The molecule forms a I:I complex with G-actin and inhibits G actin polymerization, a specificity shared with other members of the thymosin family. *In vivo* experiments with leucocytes, have indicated that Thymosin β 4 is in fact the main G actin sequestering molecule (Cassimeris L. 1992, J. Cell Biol. 119, 1261-1270). Over expression of the molecule in permanent cell lines, causes the cells to spread out more fully and to adhere more strongly. This observation suggested that Thymosin β 4 may also act as an anti apoptotic mediator (Niu, M., et al, 2000, Cell Adhes. Commun. 7, 311-320).

The role of Thymosin β 4 in the development of an atherosclerotic plaque is totally unknown. The functional implication of this molecule in the progression of the disease may be multiple.

Different possibilities, but not limited to, are described in the following :
- First, it has recently been shown that agents that disrupt the actin cytoskeleton organization including cytochalasin B, myosin light chain phosphatase, myosin light chain kinase inhibitors and simvastatin, up regulate endothelial cell Nitric Oxide Synthase (eNOS ) (Liao J.K. US patent 6,423,751). It is well established that eNOS activity is a major component of the atherogenic process (O'Driscoll G. et al, Circulation, 95, 1126-1131 ). Endothelial cells derived NO inhibits pro-atherogenic components including oxidative modification of LDL and adhesion of monocytes ( Cox D.A. and Cohen M.L.,1996, Pharm. Rev., 48, 3-19; Tsa P.S. et al, 1994, Circulation, 89, 2176-2182).

Therefore, as a regulator of G actin polymerization, Thymosin β 4 may be involved in the up regulation of eNOS and may function either as an anti or a pro atherosclerotic molecule.
- Second, survival and cell death machineries are both induced upon stimulation of endothelial cells with oxidized LDL and other stress agents. In vitro and in vivo studies in animal models or cell culture have indeed shown that endothelial cells apoptosis is initiated at sites that are prone to atherosclerosis and further development of atherosclerotic lesions, correlates with apoptosis and cell death (Isner, M et al , 1995, Circulation, 91 , 270-2711; Claise C, et al, 1999, Atherosclerosis, 147, 95-104; Dimmeler J, et al, 1997,Circulation, 95, 1760-1763). Down stream effectors of apoptosis, such as p38 MAP kinase, p53 and capsases are induced upon exposure to oxLDL and stress factors (Jing Q et al , 1999, Circ. Res., 84, 831-839; Napoli C et al. 2000, Faseb J., 14, 1996-2007; Xiuwu Zhang MD et al, 2001, Circulation, 104, 2762 - 2771). Concomitantly, oxLDL can stimulate the expression of the Zn finger transcriptional factor ATF3 and the Integrin Linked Kinase (Nawa T et al , Atherosclerosis, 2002, 161, 281-291 ; Kawauchi J et al , JBC 2002 In press). Both proteins are expressed in atherosclerotic lesions, correlate with the presence of dead cells and have been shown to regulate p38, p53 and capsase apoptotic activities. Therefore, initiation of atherosclerosis may be the result of a conflicting unbalance between apoptosis and survival, leading to vascular injury. Suppression of p38 activity and other effectors of the apoptotic machinery may constitute a feed back mechanism to protect the endothelium against oxLDL induced injury. Delineating the mechanisms that control the balance between survival and apoptosis may therefore be a fruitful approach for the discovery a new therapeutic windows and new products. Endothelial cells survival is maintained by contact to extra cellular matrix. In the absence of adhesion, endothelial cells rapidly undergo apoptosis, a phenomenon called anoikis. Integrin mediated signals are required to maintain endothelial cells integrity and reduce the sensitivity to stress. Adhesion involves focal plaque formation, activation of ILK and is probably essential in maintaining an anti atherogenic status. G actin polymerization-depolymerization is a major reaction that control cell spreading and proliferation. Therefore, Thymosin β 4 may stimulate a more complete and stronger spreading and adhesion of endothelial cells at sites of vascular lesions. Thymosin β 4 may thus act as a survival effector and prevent endothelial cells from apoptosis and cell death.
- Third, It was shown that growth factor such as the Hepatocyte Growth Factor (HGF) can up regulate the expression of Thymosin β 4 in human umbilical vein endothelial cells (Oh,I, et al Biochem., Biophys., Res. Commun. 2002, 16, 296 (2):401 ). HGF can stimulate the invasiveness of monocytes at sites of atherosclerosis and was shown to expressed in atherosclerotic plaques ( Beilmann M. 2000, Blood, 95, 3664-3669). Thymosin β 4 may thus be involved in monocyte macrophage and lymphocyte adhesion and migration at site of atherosclerosis thus contributing to plaque growth and instability.

In summary, the present invention describes for the first time a differential expression of Thymosin β 4 in an early and advanced atherosclerotic lesion. Thymosin β 4 may be considered for its development as an anti atherosclerotic target. The invention, therefore includes methods and composition for the treatment of atherosclerosis and its clinical complications by controlling Thymosin β 4 activity. The invention includes the control of the progression, erosion, and regression of an atherosclerotic plaque.

### 5) Sphingomyelinase (EC 3.1.4.12), Acide Ceramidase (EC 3.5.1.23), Sphingosine phosphate liase (EC 4.1.2.27) UDP-glucose ceramide glucosyl tranferase (EC 2.4.1.80)

Sphingomyelinase, acide ceramidase, UDP-gluclose ceramide glycosyl transferase and sphingosine phasphate liase are all important enzymes of the ceramide and sphingolipids metabolisms. The present invention indicates that these enzymes are up regulated at the level of transcription during the progression of an atherosclerotic plaque. The role the ceramide and sphingolipids patways in the process of atherosclerosis is totally unknown.

The enzyme sphingomyelinase catalyzes the hydrolysis of sphingomyelin to ceramide and choline phosphate. Different sphingomyelinase have been identified which can be separated into mitochondrial, lysosomal, cytosolic and secreted enzymes. Different and opposite functions have been ascribed to sphyngomyelinase. A role in cholesterol transfert from lysosome to the membrane has been found (Leventhal et al 2001, J. Biol. Chem. 276, 44976-44983). Uptake of oxidized LDL inhibits Lysosomal sphingomyelinase and causes accumulation of unesterified cholesterol in permanent cell line (Maor et al , 1995, ATVB, 15, 1378-1387). On the other hand, extracellular sphingomyelinase converts lipoproteins into potent atherogenic aggregated LDL (Marathe et al , 2000, ATVB, 20, 2607-2613).

Alternatively, the production and accumulation of ceramide and sphingolipid derivatives may have different consequences during the progression of a plaque. Ceramide is an important messenger of apoptosis and cell proliferation (Mathias S. et al, 1988 Biochem J. 335, 465-480) and elevated levels of ceramide in post-mortem samples of plaques in patients who died of atherosclerosis have been reported ( Schissel S L et al, 1996, J. Clin. Invest. 98, 1455-1464).

Thus activation of the ceramide pathways at the transcriptional level, may have a direct consequence on the accumulation of macrophages and foam cells at sites of atherosclerosis. Therefore, ceramide accumulation may constitute a high risk factor for plaque instability and erosion.

Alternatively, ceramide glycosil transferase catalyzes the formation of glucosylceramide An ecess production of glucosylceramide may then be responsible for an excessive accumulation of second messengers like gangliosides or globosides.

Thus the present invention identifies the sphingomyelinase/ceramide/ceramide glucosyl transferase as a potential target pathway for controlling the progression of an atherosclerotic plaque.

### 6) CD163

CD163 is an inducible member of the scavenger receptor family (Law SK et al 1993, Eur. J. Immunol. 23, 2320-2325). This receptor is induced in CD14 positive macrophages by glucocorticoids and interleukin 10. and this induction is at least in part due to increased levels of RNA and protein (patent , WO 20010041177).

The potential role of CD163 in the process of atherosclerosis is totally unknown.

### Example 4 : Gene expression in a foam cell model

A series of primary or permanent cell lines can be used to monitor the differential expression of genes that are associated with an atherosclerotic plaque. All cells should have the capacity to incorporate Lipoprotein, modified lipoprotein including oxydized acetylated lipoproteins, Triglycerides, chilomicron and to exhibit vesicules that are characteristic of an atherosclerotic plaque associated foam cell. This includes but not limited to, U937, KG1, and THP1, HUVEC, Smooth muscle cells. In the present example, THP1 cells were used to generate an expression system which can mimic the formation of a foam cell in the plaque and can be used for a large scale screening of molecules that can inhibit or control the formation of a foam cell, via the control of the expression of at least one protein

### 1) Cell culture

The THP-1 cell line from the European Collection of Cell Cultures, ( ECACC, Wilshire, UK) were selected to generate a cellular model that mimics the differentiation and the growth of a foam cell. Typically, the cells (5.10⁵ cells/ml) were maintained and grown in RPMI-1640, 10% FBS, 100 Unit/ml penicillin and 100 µg/ml streptomycin, 200 mM L-Glutamine (Biowhittaker, Verviers , Belgium) in 37°C, 5% CO2 incubator. Medium was replaced every 2-3 days.

### 2) Isolation and modification of lipoproteins

Human LDL were isolated from fresh plasma using a two steps KBr gradient ultracentrifugation (Leger et al Free Rad Res. 2002, 36, 127-142 ) LDL were dialyzed against NaCl 150 mM, sodium phosphate 10 mM, DTPA 10 µM (pH 7,4) for 24 hours. Copper oxidized LDL was prepared under sterile conditions by incubating 0.2 mg/ml of LDL with 5µM CuSO4 for 16 hours at 37°C. At the end of this incubation, oxidation was stopped by addition of BHT (40 µM final) and DTPA (100 µM final). OxLDL were extensively dialysed against NaCl 150 mM and Sodium Phosphate10 mM (pH 7,4) for 24 hours. All preparations were filtered through 0.4 µm filters.

LDL and oxLDL were extensively characterized by measuring the concentration of ApoB, total proteins, total cholesterol and vitamin E, the apparition of conjugated dienes (D0 at 234 nm) and the determination of fatty acid and oxysterol composition (see table 2).

**Table 2**

| | Protocols | LDL | oxLDL |
|---|---|---|---|
| ApoB | Immuno nephelometry | X | |
| Protein | Lowry-Maxwell | X | |
| Cholesterol | Enzymatic protocol | X | |
| Triglycerides | Chromatography | X | X |
| Fatty acids | HPLC | X | X |
| Vitamine E | Under validation | X | X |
| Oxysterols | Agarose gels | X | X |
| Electrophoretic | Spectrophotometry | X | X |
| Spectra 200-400 nm | OD | | X |
| Dienes conjugates | | | |

Finally, lipoproteins were also characterized by their electrophoretic mobility.

Labelling of OxLDL with Cyanine 3 succinimidyl ester (Amersham Pharmacia Biotech) was prepared as described (Stanton *et al*. JBC, 11992, 267, 22446-22451). At the end of the labelling procedure, Cy3-OxLDL were extensively dialysed and labelling efficiency was evaluated by measuring the absorbance at 548 nm.

### 3) Foam cell formation and RNA extraction

To induce differentiation and foam cell formation, 2.10⁶ cells/wells were plated in 6-well plates in RPMI 1640, 5% FBS supplemented with 10⁻⁷ M of phorbol 12-myristate-13-acetate (Sigma) for 24 hours at 37°C, 5% CO2. Cells were washed with 1 ml of pre-warmed medium and maintained in 2 ml of pre-warmed medium for 24 hours at 37°C, 5% CO2 to reduce specific phorbol 12-myristate-13-acetate activation. Differentiated THP-1 were incubated with low density lipoproteins (native LDL and oxLDL at 10 µg/ml and 100 µg/ml) or lipoproteins buffer in RPMI 1640, 5% FBS medium for 6 hours .

At the end of each stimulation point, cells were washed once with 2 ml of PBS, pH 7,4 and lysated by Trizol. RNA extractions were performed according the instructions of manufacturer.

Quality controls were performed in parallel on foam cell formation and cell viability. Briefly, cells were fixed with paraformaldhehyde 2% for 15 minutes at RT, washed twice with H20 and stained with Oil Red O solution to visualize intracellular lipids. Cells were counterstained with Mayer's hematoxylin for 10 minutes at RT following by fourth washing with H20. Images of foam cell formation were captured using a microscope coupled with a color CCD camera and analysis software. Finally, for each stimulation point, the viability of cells was superior to 95% after Trypan blue exclusion.

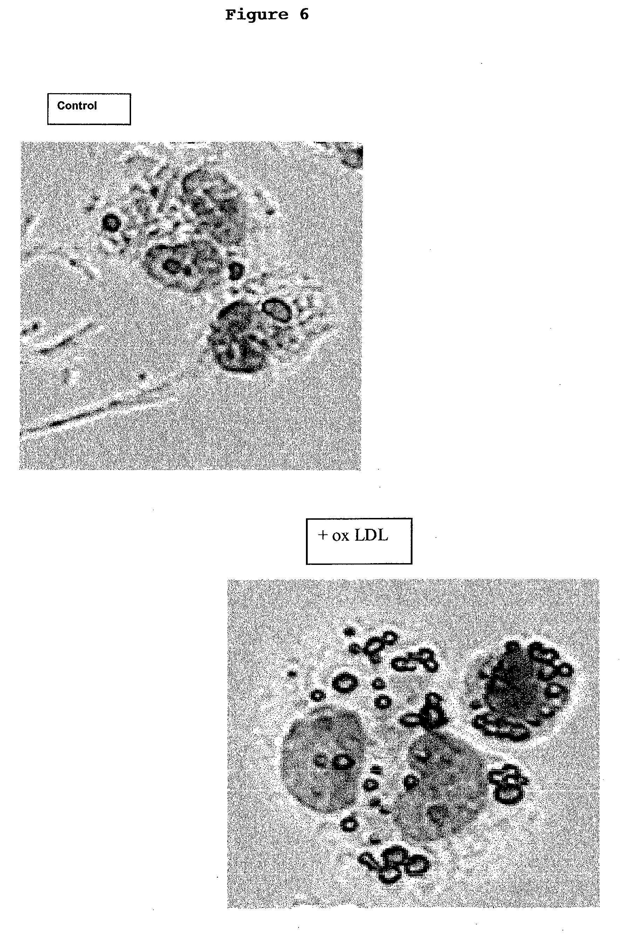

The figure 6 illustrates the uptake of oxLDL and the formation of foam cells loaded with lipid vesicules

### 4) Inhibition of lipoprotein uptake

To induce differentiation, 8.5 10⁴ cells/wells were plated in 96-well plates in culture medium supplemented with 10⁻⁷ M of phorbol 12-myristate-13-acetate (Sigma) for 24 hours at 37°C,

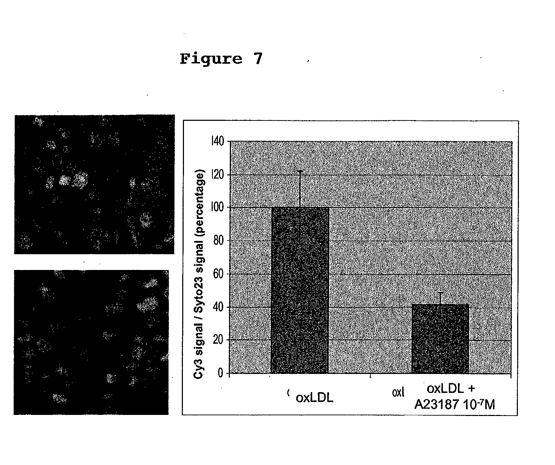

5% CO2. Cells were washed with 200 µl of pre-warmed medium. PMA-differentiated THP-1 were incubated with Cy3 labelled oxLDL (30 µg/ml) in the presence or in the absence of specific inhibitor. In the example given in figure 7, A23187 was used as a test compound time. Cells were washed twice with PBS and nucleus were counsterstained with 2.5 µM Syto 23 for 20 minutes, at RT. After washing twice in PBS, images of Cy-3 oxLDL uptake were captured using a fluoresecence microscope coupled with a CCD camera. Each image was analysed and quantified using QFluoro Software (Leica).

### 5) RNA and cDNA preparation

10⁶ cells are extracted with 1 ml Trizol (Invitrogen) following manufacturer 's instruction. RNA are resuspended in 20µl RNase DNAse free H20. cDNA where prepared as previously described (Chevillard et coll 1996) briefly 1µg RNA was reverse-transcribed using random hexamers (PdN6 Roche Diagnostics) and 1/100e of the cDNA was used in each PCR reaction (50µl final volume). PCR was performed using SYBR Green PCR or Taqman Core reagent (Applied Biosystems France), on ABI PRISM 7000 sequence detector apparatus and analysed with the dedicaced software. PCR cycles consisted of an initial step of UNG amperase at 50°C for 2 min and an initial denaturation step at 95°C for 10 min followed by 40 cycles of denaturation at 95°C for 10s and annealing-elongation at 60°C for 1min. MgCl2 concentrations were optimized for each primer set in order to minimize primer dimer formation and to reach the best amplification yield. For each amplification, the Ct value, representing the cycle at which a significant fluorescent signal is first detected, was measured. In a given sample, signals obtained for each gene were normalized to the signal obtained for a housekeeping gene (beta actin or GAPDH or beta 2 microglobulin) thus taking account of any variability in the initial concentration and quality of RNA. Finally, relative quantitation of gene expression was determined by reference to a calibration curve obtained from serial dilutions of RNA prepared from control samples expressing target gene at a high level and handled concomitantly with each RT-PCR reaction. Results were considered if the corresponding standard curve was perfectly linear, with an exponential growth of PCR products and if the level of expression of the sample was in the same range as those obtained for the standard curve.

RNA quality controls and concentration measurements were done with a bioanalyzer 2100 apparatus (Agilent, France). RNA ladder 6000 (Ambion UK) is used as a reference for quantification. Total RNA are analysed with the RNA nano labchip kit (Agilent France) For total RNA a ratio of 1 minimum between 28/18S is considered as acceptable.

### 6) PCR primer design

PCR primers and taqman probes were designed with the help of primer express 2.0 software (Applied Biosystem). Primers were chosen spaning exons junction when the genomic sequence was known. The specificity of primers was checked after alignement with FASTA software in Genbank and after amplification PCR products were checked on a 2% agarose gel electrophoresis.

Chevillard, S.; Pouillart, P.; Beldjord, C., Asselain, B., Beuzeboc, P. ; Magdalenat, H. ; Vielh, P. (1996) Sequential assesment of multidrug resistance phenotype and measurement of S-phase fraction as preditictive markers of breast cancer response to neoadjuvant chemotherapy (Cancer, 77, 292-300).

## Claims

1. A method for monitoring the growth, erosion, rupture or stability of an atherosclerotic plaque comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

2. The method of claim 1, wherein said analysis is carried out in human or animal cells, tissue sections or animal models.

3. A diagnostic method of artherosclerosis or cardiovascular disorders relating to the atherosclerotic plaque in a biological sample of a subject comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

4. The method of claim 3, wherein said analysis is carried out in human cells or tissue sections.

5. The method of any of claims 1 to 4, wherein the analysis is performed at the mRNA or protein level.

6. The method of claims 1 or 3, which comprises :
- providing a plurality of different ligands in the form of an array on a solid surface, said different ligands being complementary to different segments of at least one protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, or being complementary to different segments of at least one gene coding said proteins,
- applying a sample solution potentially containing the targets of the ligands to the array of ligands under conditions which allow the interaction of said ligands and its target, and
- measuring the interactions of the targets with the different ligands of the array.

7. The method of claim 6, wherein the ligands are nucleic acid probes and the sample contains target nucleic acids in order to measure the hybridation of the probes with the target nucleic acids.

8. The method of claim 7, wherein the nucleic acid probes are oligonucleotides.

9. The method of claim 8, wherein the array comprises 2 to about 200 oligonucleotides localized in discrete location per square centimeter on the solid surface.

10. The method according to any of claims 6 to 9, wherein the sample is from a patient developing artherosclerotic plaque.

11. Method of screening compounds useful for the treatment of artherosclerosis or cardiovascular disorders relating to the atherosclerotic plaque comprising the analysis of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, in the presence of a test compound.

12. The method of claim 11, wherein said analysis is carried out in human or animal cells, tissue sections or animal models.

13. The method of any of claims 11 and 12, wherein the analysis is performed at the mRNA or protein level.

14. The method of claim 13, wherein the analysis is performed on a solid support.

15. The method of claim 11, which comprises :
- providing a plurality of different ligands in the form of an array on a solid surface, said different ligands consisting of all or part of at least one protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, ,
- applying a solution containing a test compound to the array of ligands, and
- measuring the interaction, such as the binding, of the test compound with the different ligands of the array.

16. The method according to any of claims 1 to 15, wherein the test compound is a protein or molecule of small molecular weight.

17. Method of screening compounds useful for the treatment of artherosclerosis or cardiovascular disorders relating to the atherosclerotic plaque according to any of claims 11 to 13 comprising :
- providing an assay for at least one protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, in the presence of a test compound.
- contacting said assay with a test compound, and
- measuring the action of the test compound on the said protein in the assay.

18. The method of any of claims 1 to 17, wherein the analysis comprises the measure of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, and the comparison of said measure with the normal expression of said protein in early and advanced atherosclerotic plaques containing macrophages, under hyperlypidemic conditions and in the absence of high levels of blood glucose and insulin.

19. The method of any of claims 1 to 18, wherein the analysis comprises the measure of the differential expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, and the comparison of said measure with the expression of reference genes that are representative of an atherosclerotic plaque.

20. The method of claim 19, wherein said references gene include membrane associated genes such as CD68, CD36 which are both markers of the macrophage lineage; PECAM 1, a marker for endothelial cells; markers of the inflammatory response such as TLR4, HSP60 and HSP70, Galectin 3 and IL1-R; markers of the oxidative stress including HIF-1 and Paraoxanase 3, metabolic marker such as NADH dehydrogenase; lipoprotein receptors such as LDL-R and VLDL-R.

21. The use of a compound modulating the expression of at least one gene coding a protein chosen among Stearoyl CoA deasturase, Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163, or modulating the activity of said at least one protein for the preparation of a pharmaceutical composition useful for preventing and/or treating artherosclerosis or cardiovascular disorders relating to the atherosclerotic plaque.
